# EUROPEAN PATENT APPLICATION

(11) **EP 3 750 906 A1**
(43) Date of publication of application: **16.12.2020**
(21) Application number: 19750572.0
(22) Date of filing: 08.02.2019
(51) Int. Cl.: C07H 19/20

(54) **PRODUCTION METHOD AND PRODUCTION INTERMEDIATE FOR GUANOSINE-3',5'-BISDIPHOSPHATE**

(30) Priority: 09.02.2018 WO PCT/JP2018/004749
(71) Applicant: Tokyo Institute of Technology, Tokyo 152-8550 (JP)
(72) Inventor: SEIO, Kohji, Tokyo 152-8550 (JP); MASAKI, Yoshiaki, Tokyo 152-8550 (JP); OHNO, Kentaro, Tokyo 152-8550 (JP); TOMORI, Takahito, Tokyo 152-8550 (JP)
(74) Representative: Ricker, Mathias
(86) International application number: PCT/JP2019/004725
(87) International publication number: WO 2019/156240

(57) **Abstract**

The present invention relates to a production method and a production intermediate for guanosine-3',5'-bisdiphosphate.

## Description

### FIELD

The present invention relates to a production method and a production intermediate for guanosine-3',5'-bisdiphosphate (hereinafter also referred to as "ppGpp").

### BACKGROUND

Microbes such as bacteria have a mechanism for controlling an in vivo reaction as a way to defuse crisis situations of life due to an amino-acid deficiency state. Such a mechanism is called a stringent response, and it is known that a central molecule of the stringent response mechanism is ppGpp.

The synthesis of ppGpp in microbes is catalyzed by two enzymes called RelA and SpoT. These enzymes synthesize ppGpp by transferring a pyrophosphoric acid at a βγ position of ATP to GTP or a 3' terminal of GDP. The biosynthesized ppGpp inhibits transcription, translation, replication, and biosynthesis of GTP in the microbes and functions to suppress wasteful energy consumption until the amino-acid deficiency state is eliminated. Since amino-acid synthases are not inhibited, amino acids are gradually stored in the microbes. When the starvation state is broken, the synthesis of ppGpp is suppressed, and the in vivo reaction is restored. The RelA protein only has synthetic activity of ppGpp, whereas the SpoT protein has synthetic activity and decomposition activity of ppGpp and plays a role in degradation of ppGpp after the starvation state is broken.

Recently, it has become obvious that ppGpp functions also in plant growth (NPL 1 and NPL 2). The action of ppGpp in higher organisms is an interesting object that should be studied in the future.

Although ppGpp is commercially available, the supply of ppGpp is mainly performed by enzyme synthesis. Moreover, a method of producing ppGpp by chemical synthesis has been reported (NPL 3).

### [CITATION LIST]

### [NON PATENT LITERATURE]

[NPL 1] Maekawa M. et al., Nat Plants. 2015, 1, 15167
[NPL 2] Ihara Y. et al., Plant Signal Behav., 2016; 11(2): e1132966
[NPL 3] Simoncsits A et al., Biochim. Biophys. Acta, 340 (1974) pp. 509-515

### SUMMARY

### [TECHNICAL PROBLEM]

In studying the action of ppGpp in organisms, an inexpensive and stable supply of ppGpp is necessary. However, ppGpp commercially available today is extremely expensive, thereby contributing to the inhibition of the progress of this study.

The currently mainstream ppGpp synthesis by enzymes is not suitable for large-scale synthesis. Moreover, the chemical synthesis method of ppGpp described in NPL 3 includes purification by ion-exchange chromatography in the purification step. Since the elution is performed using a high-viscosity aqueous solvent in the ion-exchange chromatography, the flow rate is low. Moreover, since the elution is performed using a buffer solution, desalting treatment needs to be performed after the purification. Furthermore, in the method of producing ppGpp from guanosine-3',5'-bisphosphate described in NPL 3, the total yield is extremely low, about 5%. Therefore, the method of NPL 3 is low in efficiency and is not suitable for large-scale synthesis of ppGpp.

It is an object of the present invention to provide a method capable of efficiently producing ppGpp.

### [SOLUTION TO PROBLEM]

The present inventors made extensive research in view of the above-described object, found a production intermediate suitable for efficient production of ppGpp, and achieved the present invention.

The present invention encompasses the following <1> to <9>.
<1> A production method of a compound or a salt thereof represented by formula (I): 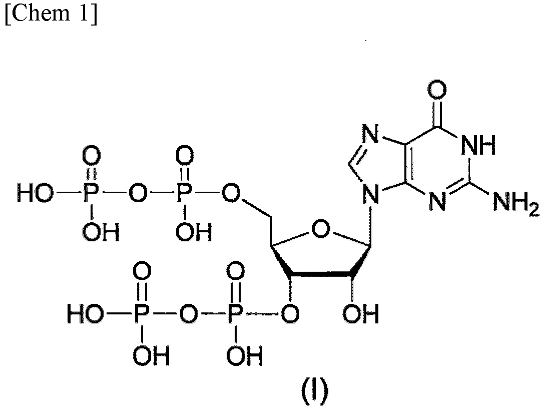 , the production method comprising:
   a step of removing Si(R¹)(R²)(R³), and R⁴ and R⁵ from a compound or a salt thereof represented by formula (II): 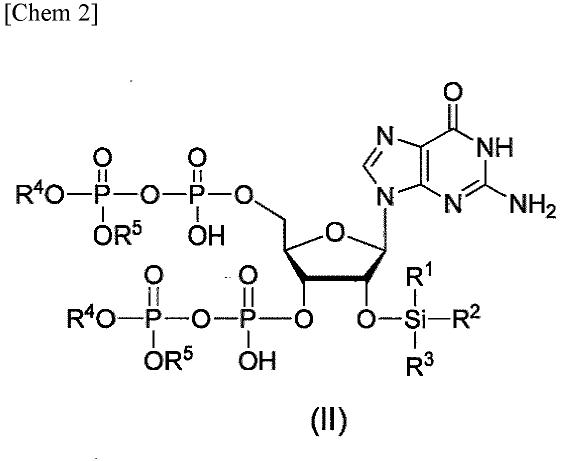 wherein
   R¹ to R³ each independently represents C₁₋₁₀ alkyl or C₄₋₁₀ cycloalkyl, and
   R⁴ and R⁵ each independently represents a protective group.
<2> The production method according to <1>, wherein the step of removing Si(R¹)(R²)(R³), and R⁴ and R⁵ comprises:
   a step of removing R⁴ and R⁵ from the compound or a salt thereof represented by formula (II) to obtain a compound or a salt thereof represented by formula (III): 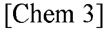 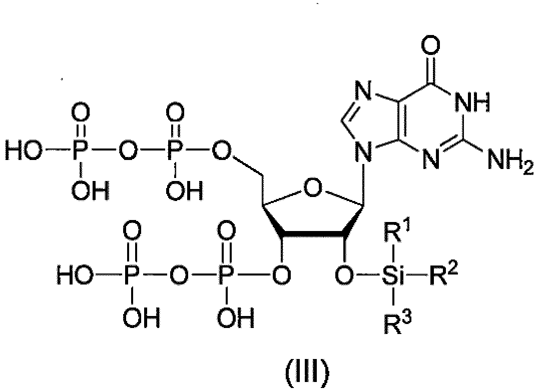 wherein
   R¹ to R³ each independently represents C₁₋₁₀ alkyl or C₄₋₁₀ cycloalkyl; and
   a step of removing Si(R¹)(R²)(R³) from the compound or a salt thereof represented by formula (III) to obtain the compound or a salt thereof represented by formula (I).
<3> The production method according to <1> or <2>, further comprising:
   a step of reacting a compound or a salt thereof represented by formula (IV): 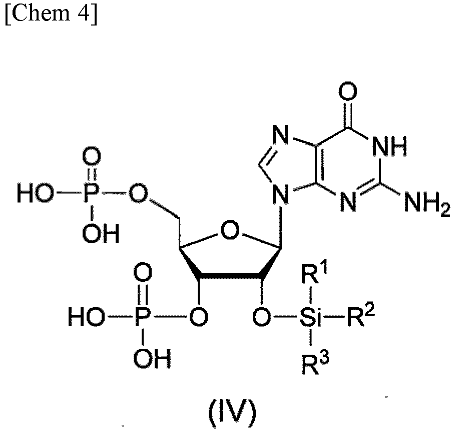 wherein
   R¹ to R³ each independently represents C₁₋₁₀ alkyl or C₄₋₁₀ cycloalkyl, with a phosphoramidite represented by formula (V): 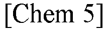 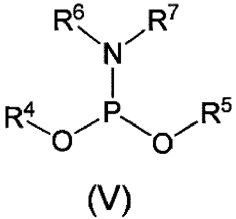 wherein
   R⁴ and R⁵ each independently represents a protective group, and
   R⁶ and R⁷ each independently represents C₁₋₁₀ alkyl or C₄₋₁₀ cycloalkyl or forms a pyrrolidine ring, a piperidine ring, or a morpholine ring with an adjacent nitrogen atom,
   in the presence of an activator to obtain a compound or a salt thereof represented by formula (VI): 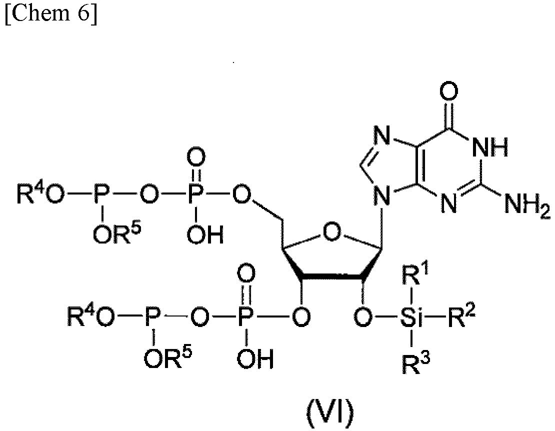 and
   a step of oxidizing the compound or a salt thereof represented by formula (VI) to obtain the compound or a salt thereof represented by formula (II).
<4> The production method according to any one of <1> to <3>, wherein R¹ and R² each represents methyl, R³ represents tert-butyl, and R⁴ and R⁵ each represents benzyl.
<5> The production method according to any one of <1> to <4>, further comprising:
   a step of reacting a compound or a salt thereof represented by formula (VII): 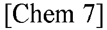 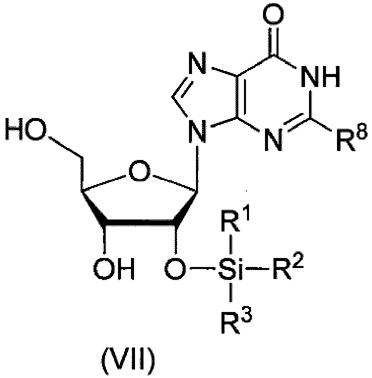 wherein
   R¹ to R³ each independently represents C₁₋₁₀ alkyl or C₄₋₁₀ cycloalkyl,
   R⁸ represents NHR⁹ or N=CH-N(R¹⁰)(R¹¹),
   R⁹ represents a protective group that can be removed under a basic condition, a protective group that can be removed under a reductive condition, or a protective group that can be removed by a light reaction, and
   R¹⁰ and R¹¹ each independently represents C₁₋₁₀ alkyl or C₄₋₁₀ cycloalkyl, with a phosphoramidite represented by formula (VIII): 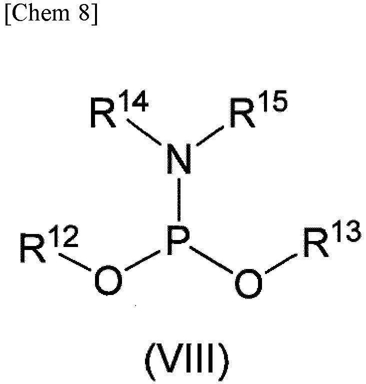 wherein
      R¹² and R¹³ each independently represents a protective group that can be removed under a basic condition, a protective group that can be removed under a reductive condition, or a protective group that can be removed by a light reaction, and
      R¹⁴ and R¹⁵ each independently represents C₁₋₁₀ alkyl or C₄₋₁₀ cycloalkyl or forms a pyrrolidine ring, a piperidine ring, or a morpholine ring with an adjacent nitrogen atom,
      in the presence of an activator to obtain a compound or a salt thereof represented by formula (IX): 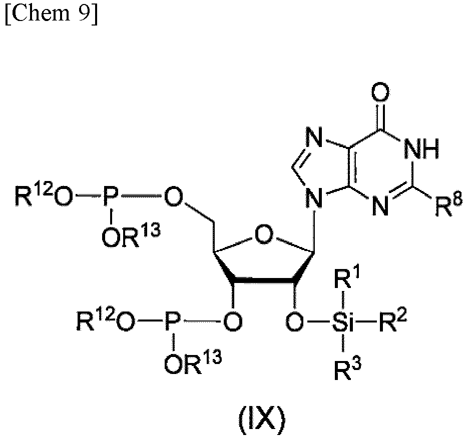
      a step of oxidizing the compound represented by formula (IX) to obtain a compound or a salt thereof represented by formula (X): 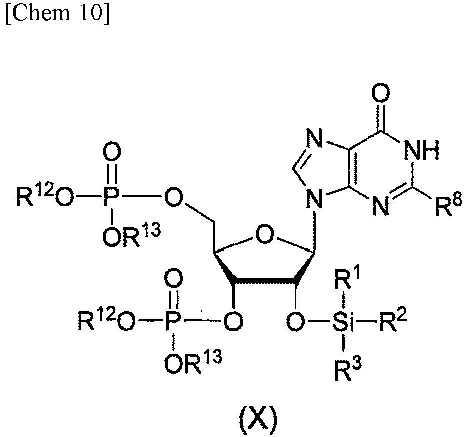 and
      a step of removing R⁹ or CH-N(R¹⁰)(R¹¹), and R¹² and R¹³ from the compound represented by formula (X) to obtain the compound or a salt thereof represented by formula (IV).
<6> The production method according to <5>, wherein R¹² and R¹³ each represents 2-cyanoethyl, and R⁸ represents N=CH-N(CH₃)₂.
<7> A compound or a salt thereof represented by formula (II): 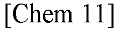 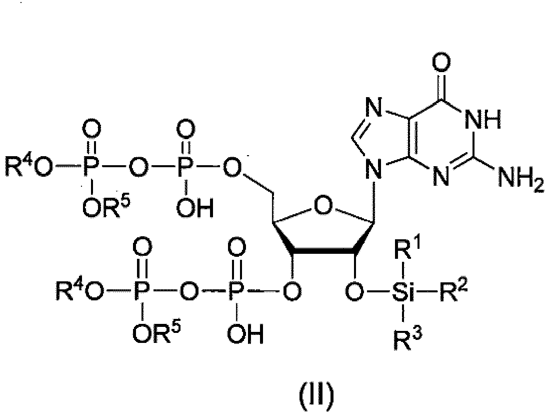 wherein
   R¹ to R³ each independently represents C₁₋₁₀ alkyl or C₄₋₁₀ cycloalkyl, and
   R⁴ and R⁵ each independently represents a protective group.
<8> A compound or a salt thereof represented by formula (III): 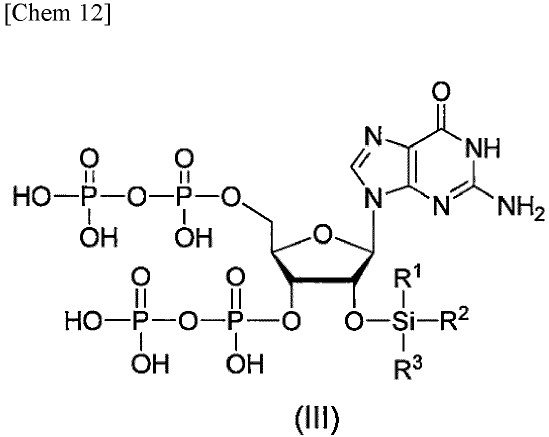 wherein
   R¹ to R³ each independently represents C₁₋₁₀ alkyl or C₄₋₁₀ cycloalkyl.
<9> A compound or a salt thereof represented by formula (IV): 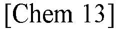 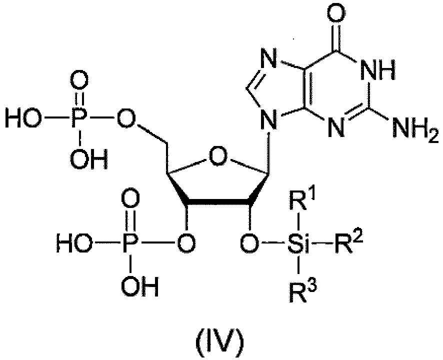 wherein
   R¹ to R³ each independently represents C₁₋₁₀ alkyl or C₄₋₁₀ cycloalkyl.

### [ADVANTAGEOUS EFFECTS OF INVENTION]

According to the present invention, ppGpp can be efficiently produced.

### DESCRIPTION OF EMBODIMENTS

### <<Definitions>>

In the present description, "C₁₋₁₀ alkyl" refers to a straight-chain or branched-chain alkyl group having 1 to 10 carbon atoms. Examples of the C₁₋₁₀ alkyl include C₁₋₇ alkyl and C₁₋₅ alkyl. Examples of the C₁₋₁₀ alkyl include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, heptyl, octyl, nonyl, and decyl.

In the present description, "C₄₋₁₀ cycloalkyl" refers to a cyclic alkyl group having 4 to 10 carbon atoms. Examples of the C₄₋₁₀ cycloalkyl include C₄₋₈ cycloalkyl and C₄₋₆ cycloalkyl. Examples of the C₄₋₁₀ cycloalkyl include cyclobutyl, cyclopentyl, cyclohexyl, cycloheptyl, cyclooctyl, cyclononyl, and cyclodecyl.

In the present description, examples of a "salt" include an acid addition salt, an alkali metal salt, an alkaline-earth metal salt, an ammonium salt, and an amine salt. Examples of the acid addition salt include inorganic acid salts such as a hydrochloride, a hydrobromide, a hydroiodide, a hydrosulfate, a phosphate, and a nitrate, and organic acid salts such as an acetate, a lactate, a tartrate, a benzoate, a citrate, a methanesulfonate, an ethanesulfonate, a trifluoroacetate, a benzenesulfonate, a toluenesulfonate, an isethionate, a glucuronate, and a gluconate. Examples of the alkali metal salt include a potassium salt and a sodium salt. Examples of the alkaline-earth metal salt include a calcium salt and a magnesium salt. Examples of the ammonium salt include a tetramethylammonium salt. Examples of the amine salt include a triethylamine salt, a diazabicyclo-undecene (DBU) salt, a methylamine salt, a dimethylamine salt, a cyclopentylamine salt, a benzylamine salt, a phenethylamine salt, a piperidine salt, a monoethanolamine salt, a diethanolamine salt, a tris(hydroxymethyl)aminomethane salt, a lysine salt, an arginine salt, and a N-methyl-D-glucamine salt. According to a method well known to those skilled in the art, ppGpp and a production intermediate thereof produced by the method of the invention of the present application can be converted into a salt.

### <<Production Method of ppGpp>>

One mode of the present invention is a production method of a compound (ppGpp) or a salt thereof represented by formula (I): 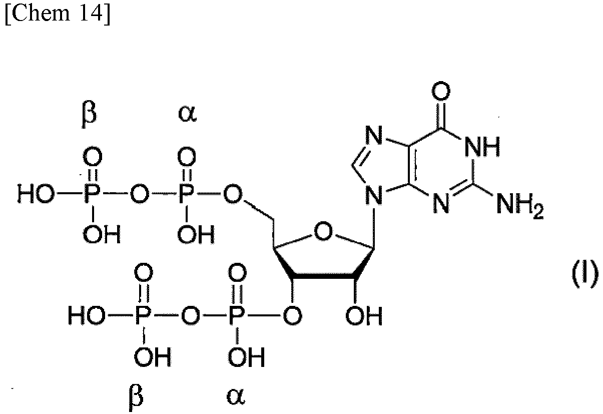 , the production method comprising:
a step of removing Si(R¹)(R²)(R³), and R⁴ and R⁵ from a compound or a salt thereof represented by formula (II): 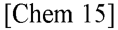 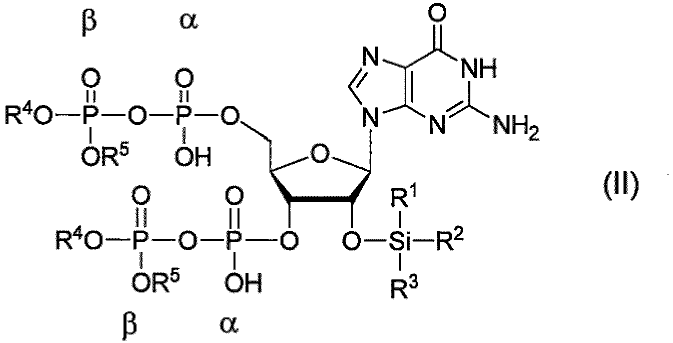 wherein
R¹ to R³ each independently represents C₁₋₁₀ alkyl or C₄₋₁₀ cycloalkyl, and
R⁴ and R⁵ each independently represents a protective group. Hereinafter, the production method will be referred to as a "production method of ppGpp of the present invention".

In order to achieve efficient synthesis of ppGpp, preferably, purification of ppGpp and a synthetic intermediate thereof is easy. Since a β-phosphoric acid on the side of a 5'-hydroxyl group and a β-phosphoric acid on the side of a 5'-hydroxyl group are protected, the compound represented by formula (II) has higher lipid solubility compared to a compound in which a phosphoric acid is not protected. Moreover, as a protective group of a 2'-hydroxyl group, a silyl-type protective group having high lipid solubility is introduced. Therefore, the compound represented by formula (II) can be efficiently purified by a method suitable for large-scale synthesis, such as silica gel column chromatography using an organic solvent and reprecipitation. Moreover, the silyl-type protective group of the 2'-hydroxyl group can be removed under a mild acid condition due to adjacent group contribution of the phosphoric acids introduced into the 3'-hydroxyl group.

From the viewpoint of easiness of purification and deprotection and the like, the protective group of the 2'-hydroxyl group is preferably tert-butyldimethylsilyl (TBDMS) (R¹ and R²: methyl, R³: tert-butyl), triethylsilyl (R¹, R², and R³: ethyl), triisopropylsilyl (R¹, R², and R³: isopropyl), or the like, and more preferably tert-butyldimethylsilyl.

Although R⁴ and R⁵ are not particularly limited, for example, a protective group that can be removed under an acid condition, a protective group that can be removed under a basic condition, a protective group that can be removed under a reductive condition, or a protective group that can be removed by a light reaction can be used. The protective group that can be removed under a basic condition, the protective group that can be removed under a reductive condition, or the protective group that can be removed by a light reaction is preferable. Examples of the protective group that can be removed under a basic condition include 2-cyanoethyl, a fluorenylmethyl group, and 2-(4-nitrophenyl)ethyl. Examples of the protective group that can be removed under a reductive condition include benzyl, 4-methoxybenzyl, and naphthylmethyl. Examples of the protective group that can be removed by a light reaction include a 2-nitrobenzyl group and 2-nitrophenylethyl. From the viewpoint of easiness of purification and deprotection and the like, benzyl is preferable.

The order in which Si(R¹)(R²)(R³), and R⁴ and R⁵ are removed from the compound or a salt thereof represented by formula (II) can be appropriately selected depending on the properties of the protective group to be used. For example, the removal of Si(R¹)(R²)(R³) and the removal of R⁴ and R⁵ may be performed at the same time. Alternatively, R⁴ and R⁵ may be removed after removing Si(R¹)(R²)(R³). Alternatively, Si(R¹)(R²)(R³) may be removed after removing R⁴ and R⁵ and obtaining a compound or a salt thereof represented by the following formula (III): 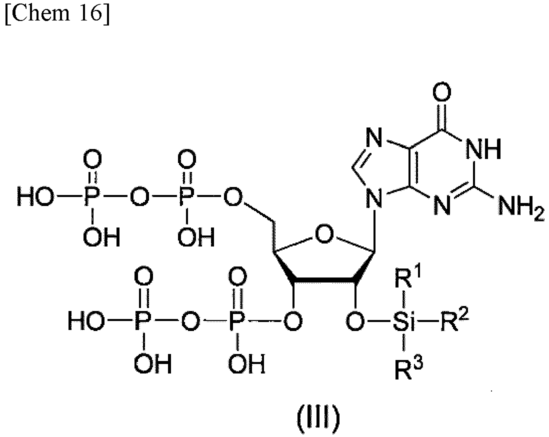 wherein
R¹ to R³ each independently represents C₁₋₁₀ alkyl or C₄₋₁₀ cycloalkyl.

The condition for removing R⁴ and R⁵ can be appropriately selected depending on the type of the protective group to be used. Si(R¹)(R²)(R³) can be removed by neutral aqueous organic solvent treatment, acid treatment (for example, treatment at a pH of 3 or more and less than 7), or fluoride ion treatment, or by a combination of the treatment. In the aqueous solvent treatment, as an organic solvent in the aqueous organic solvent, any organic solvent can be used as long as it combines with water. A polar organic solvent is preferable. Examples of the polar solvent include a lower alcohol (for example, methanol, ethanol), tetrahydrofuran, dioxane, dimethylformamide, dimethylsulfoxide, acetonitrile, acetone, and hexamethylphosphoric triamide (HMPA). The aqueous organic solvent treatment can be performed at from 20°C to 60°C, for example. Although not particularly limited, the acid treatment can be performed using an acetic acid aqueous solution, for example. Although not particularly limited, the fluoride ion treatment can be performed using, for example, tetrabutylammonium fluoride (TBAF), tris(dimethylamino)sulfonium difluorotrimethylsilicate (TASF), HF-pyridine complex, HF-triethylamine complex, or the like. Preferably, the removal of Si(R¹)(R²)(R³) is performed by the neutral aqueous organic solvent treatment or the acid treatment.

In a preferred embodiment, in formula (II), R¹ and R² each represents methyl, R³ represents tert-butyl, and R⁴ and R⁵ each represents benzyl. When these protective groups are used, a by-product generated after the removal can be easily separated from ppGpp, and thus, ppGpp can be successfully isolated. When these protective groups are used, ppGpp can be produced by removing tert-butyldimethylsilyl by the neutral aqueous organic solvent treatment or the acid treatment (for example, a pH of 3 or more and less than 7) after removing benzyl by hydrogenation in the presence of a Pd catalyst, for example.

The compound represented by formula (II) may be produced through the following steps (A) and (B):
(A) a step of reacting a compound or a salt thereof represented by formula (IV): 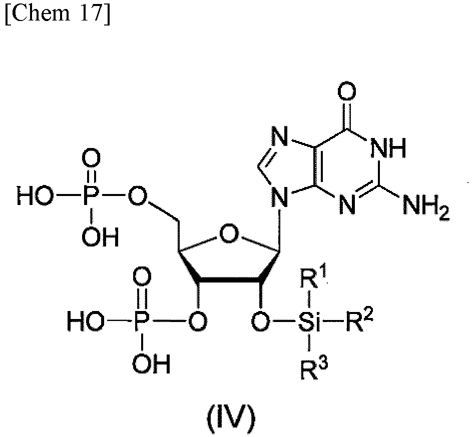 wherein
   R¹ to R³ each independently represents C₁₋₁₀ alkyl or C₄₋₁₀ cycloalkyl, with a phosphoramidite represented by formula (V): 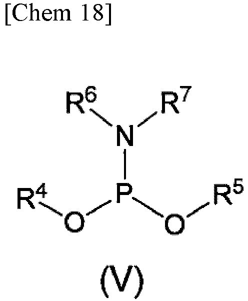 wherein
   R⁴ and R⁵ each independently represents a protective group, and
   R⁶ and R⁷ each independently represents C₁₋₁₀ alkyl or C₄₋₁₀ cycloalkyl or forms a pyrrolidine ring, a piperidine ring, or a morpholine ring with an adjacent nitrogen atom,
   in the presence of a base and an activator to obtain a compound or a salt thereof represented by formula (VI): 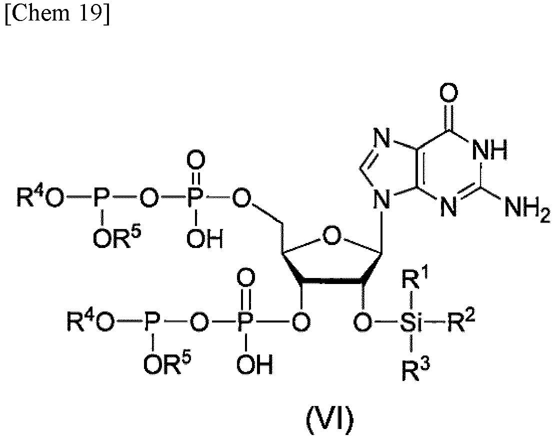 and
(B) a step of oxidizing the compound or a salt thereof represented by formula (VI).

The compound or a salt thereof represented by formula (IV) is an extremely useful starting material for efficiently producing the compound represented by formula (II).

When 3' and 5' phosphate groups of the compound represented by formula (IV) activated by being linked to a leaving group are reacted with phosphoric acid, an intramolecular cyclization reaction progresses, and the intended compound represented by formula (II) cannot be obtained. The present inventors found that a side reaction is avoided and the intended compound represented by formula (II) can be produced by reacting the 3' and 5' phosphate groups of the compound represented by formula (IV) as nucleophiles with a phosphoramidite reagent.

In the phosphoramidite represented by formula (V) used in the step (A), preferably, R⁶ and R⁷ each independently represents ethyl or isopropyl or forms a pyrrolidine ring, a piperidine ring, or a morpholine ring with an adjacent nitrogen atom. More preferably, R⁶ and R⁷ each independently represents isopropyl. The molar ratio of the compound or a salt thereof represented by formula (IV) to the phosphoramidite represented by formula (V), which are used in the step (A), is preferably from 1:2 to 1:10, and more preferably 1:4. The phosphoramidite represented by formula (V) can be produced by a method well known to those skilled in the art. Moreover, as the phosphoramidite represented by formula (V), a commercialized product may be used.

In the step (A), a base may be used. Although the base is not particularly limited as long as the reaction proceeds, examples of the base include tri(n-butyl)amine, diazabicyclo-undecene (DBU), diisopropylethylamine, triethylamine, N-methylmorpholine, N-methylpyrrolidine, N-methylpiperidine, and a mixture thereof. Tri(n-butyl)amine, DBU, or the mixture thereof is preferable. The molar ratio of the compound or a salt thereof represented by formula (IV) to the base, which are used in the step (A), is preferably from 1:2 to 1:6, and more preferably 1:4.

Although the activator used in the step (A) is not particularly limited as long as the reaction proceeds, examples of the activator include a pyridine hydrochloride; a pyridine sulfonate such as:
pyridinium triflate;
a pyridine borate such as pyridinium tetrafluoroborate;
an imidazole such as 4,5-dicyanoimidazole or 4,5-dichloroimidazole;
a tetrazole such as 5-benzyltetrazole, 5-ethylthio-1H-tetrazole, 1H-tetrazole, or 5-(3,5-bistrifluoromethylphenyl)-1H-tetrazole;
an imidazole sulfonate such as imidazolium triflate;
a benzimidazole sulfonate such as 5-nitrobenzimidazolium triflate;
a triazole sulfonate such as triazolium triflate;
aN-hydroxy triazole such as 1-hydroxybenzotriazole; a triazole;
a N-(cyanomethyl)amine borate such as N-(cyanomethyl)pyrrolidinium tetrafluoroborate;
a N-(cyanomethyl)amine sulfonate such as N-(cyanomethyl)pyrrolidinium triflate; and
a N-(cyanomethyl)amine phosphate such as N-(cyanomethyl)pyrrolidinium hexafluorophosphate. 5-Ethylthio-1H-tetrazole is preferable. The molar ratio of the phosphoramidite represented by formula (V) to the activator, which are used in the step (A), is preferably from 1:1 to 1:6, and more preferably from 1:1 to 1:5, from 1:1 to 1:4, from 1:1 to 1:3, or from 1:1 to 1:2.

Although a solvent used in the step (A) is not particularly limited as long as the reaction proceeds, examples of the solvent include N,N-dimethylformamide (DMF), acetonitrile, dichloromethane, tetrahydrofuran, and a mixed solvent thereof. The reaction temperature in the step (A) is preferably from -78°C to 30°C, and more preferably from 0°C to 30°C.

The mixing order of the reagents in the reaction of the step (A) is not particularly limited as long as the reaction proceeds. For example, all the reagents may be simultaneously added to and mixed in the solvent, or the reagents may be gradually added to and mixed in the solvent. Preferably, the reaction of the step (A) is performed by, after the phosphoramidite represented by formula (V) and the activator are mixed in the solvent to activate the phosphoramidite, adding the mixed solution to a solution of the compound or a salt thereof represented by formula (IV).

In the reaction of the step (B), as an oxidant, for example, iodine, tert-butylhydroperoxide, camphorsulfonyloxaziridine, and the like can be used. Iodine and tert-butylhydroperoxide are preferable.

Although a solvent used in the step (B) is not particularly limited as long as the reaction proceeds, examples of the solvent include N,N-dimethylformamide (DMF), acetonitrile, dichloromethane, pyridine, tetrahydrofuran, and a mixed solvent thereof. Although the reaction temperature in the step (B) is not particularly limited as long as the reaction proceeds, from - 78°C to 30°C is preferable, and from 0°C to 30°C is more preferable.

The reaction of the step (B) may be performed after the compound represented by formula (VI) obtained in the step (A) is purified and isolated, or may be performed by, after the reaction of the step (A) proceeds, adding the oxidant to the reaction solution.

The compound or a salt thereof represented by formula (IV) may be produced through, for example, the following steps (C), (D), and (E):
(C) a step of reacting a compound or a salt thereof represented by formula (VII): 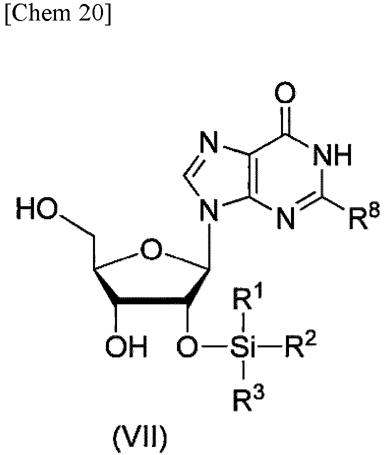 wherein
   R¹ to R³ each independently represents C₁₋₁₀ alkyl or C₄₋₁₀ cycloalkyl,
   R⁸ represents NHR⁹ or N=CH-N(R¹⁰)(R¹¹),
   R⁹ represents a protective group that can be removed under a basic condition, a protective group that can be removed under a reductive condition, or a protective group that can be removed by a light reaction, and
   R¹⁰ and R¹¹ each independently represents C₁₋₁₀ alkyl or C₄₋₁₀ cycloalkyl, with a phosphoramidite represented by formula (VIII): 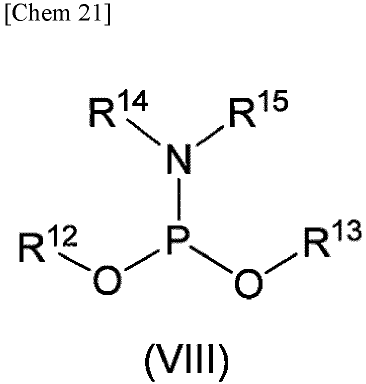 wherein
      R¹² and R¹³ each independently represents a protective group that can be removed under a basic condition, a protective group that can be removed under a reductive condition, or a protective group that can be removed by a light reaction, and
      R¹⁴ and R¹⁵ each independently represents C₁₋₁₀ alkyl or C₄₋₁₀ cycloalkyl or forms a pyrrolidine ring, a piperidine ring, or a morpholine ring with an adjacent nitrogen atom,
      in the presence of an activator to obtain a compound or a salt thereof represented by formula (IX): 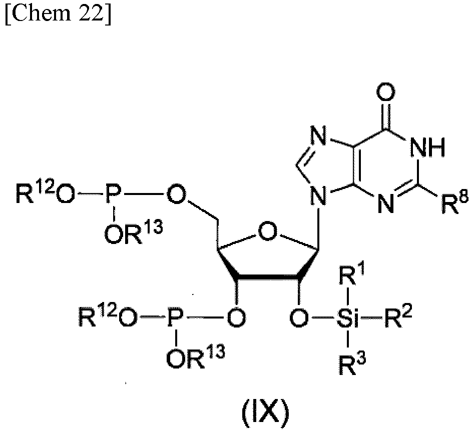
(D) a step of oxidizing the compound or a salt thereof represented by formula (IX) to obtain a compound or a salt thereof represented by formula (X): 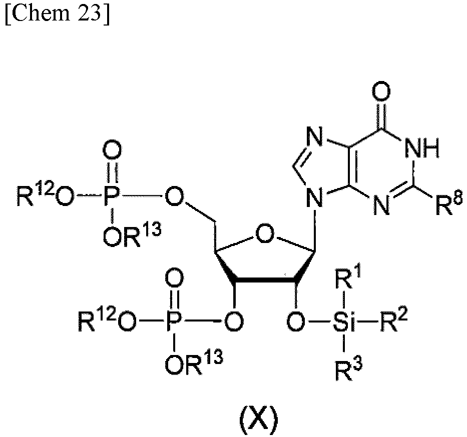 and
(E) a step of removing R⁹ or CH-N(R¹⁰)(R¹¹), and R¹² and R¹³ from the compound or a salt thereof represented by formula (X) to obtain the compound or a salt thereof represented by formula (IV).

The compound or a salt thereof represented by formula (VII) used in the step (C) can be produced by a method well known to those skilled in the art.

In formula (VII), R⁸ represents NHR⁹ or N=CH-N(R¹⁰)(R¹¹). R⁹ represents a protective group that can be removed under a basic condition (for example, acyl), a protective group that can be removed under a reductive condition (for example, benzyloxycarbonyl), or a protective group that can be removed by a light reaction (for example, 2-nitrobenzyloxycarbonyl). The protective group that can be removed under a basic condition is preferable, and acyl (for example, isobutyryl) is more preferable. Moreover, =CH-N(R¹⁰)(R¹¹) is a protective group that can be removed under a basic condition, R¹⁰ and R¹¹ each independently represents C₁₋₁₀ alkyl or C₄₋₁₀ cycloalkyl, and preferably, R¹⁰ and R¹¹ each represents methyl.

Preferably, the compound represented by formula (VII) is a compound in which R¹ and R² each represents methyl, R³ represents tert-butyl, and R⁸ represents N=CH-N(CH₃)₂.

In the phosphoramidite represented by formula (VIII) used in the step (C), preferably, R¹⁴ and R¹⁵ each independently represents ethyl or isopropyl or forms a pyrrolidine ring, a piperidine ring, or a morpholine ring with an adjacent nitrogen atom. More preferably, R¹⁴ and R¹⁵ each represents isopropyl.

In the phosphoramidite represented by formula (VIII) used in the step (C), R¹² and R¹³ is each selected from the group consisting of a protective group that can be removed under a basic condition, a protective group that can be removed under a reductive condition, and a protective group that can be removed by a light reaction. Examples of the protective group that can be removed under a basic condition include 2-cyanoethyl, a fluorenylmethyl group, and 2-(4-nitrophenyl)ethyl. Examples of the protective group that can be removed under a reductive condition include benzyl, 4-methoxybenzyl, and naphthylmethyl. Examples of the protective group that can be removed by a light reaction include a 2-nitrobenzyl group and 2-nitrophenylethyl. From the viewpoint of easiness of purification and deprotection and the like, 2-cyanoethyl is preferable.

The molar ratio of the compound or a salt thereof represented by formula (VII) to the phosphoramidite represented by formula (VIII), which are used in the step (C), is preferably from 1:2 to 1:10, and more preferably from 1:2 to 1:5, from 1:2 to 1:4, or from 1:2 to 1:3. The phosphoramidite represented by formula (VIII) can be produced by a method well known to those skilled in the art. Moreover, as the phosphoramidite represented by formula (VIII), a commercialized product may be used.

As the activator used in the step (C), the same as that used in the step (A) can be used. 1H-tetrazole is preferable. The molar ratio of the phosphoramidite represented by formula (VIII) to the activator, which are used in the step (C), is preferably from 1:1 to 1:6, and more preferably from 1:1 to 1:5, from 1:1 to 1:4, from 1:1 to 1:3, or from 1:1 to 1:2.

Although a solvent used in the step (C) is not particularly limited as long as the reaction proceeds, examples of the solvent include acetonitrile, N,N-dimethylformamide (DMF), dichloromethane, and a mixed solvent thereof. The reaction temperature in the step (C) is preferably from -78°C to 30°C, and more preferably from 0°C to 30°C.

The mixing order of the reagents in the reaction of the step (C) is not particularly limited as long as the reaction proceeds. For example, all the reagents may be simultaneously added to and mixed in the solvent, or the reagents may be gradually added to and mixed in the solvent. Preferably, the reaction of the step (C) is performed by, after the phosphoramidite represented by formula (VIII) and the activator are mixed in the solvent to activate the phosphoramidite, adding the mixed solution to a solution of the compound or a salt thereof represented by formula (VI).

In the reaction of the step (D), as an oxidant, the same as that in the step (B) can be used. Although a solvent used in the step (D) is not particularly limited as long as the reaction proceeds, examples of the solvent include N,N-dimethylformamide (DMF), acetonitrile, dichloromethane, pyridine, tetrahydrofuran, and a mixed solvent thereof. Although the reaction temperature in the step (D) is not particularly limited as long as the reaction proceeds, from - 78°C to 30°C is preferable, and from 0°C to 30°C is more preferable.

The reaction of the step (D) may be performed after the compound represented by formula (IX) obtained in the step (C) is purified and isolated, or may be performed by, after the reaction of the step (C) proceeds, adding the oxidant to the reaction solution.

In the reaction of the step (E), the order in which R⁹ or CH-N(R¹⁰)(R¹¹), and R¹² and R¹³ are removed from the compound represented by formula (X) can be appropriately selected depending on the properties of the protective group to be used. For example, the removal of R⁹ or CH-N(R¹⁰)(R¹¹), and the removal of R¹² and R¹³ from the compound represented by formula (X) may be performed at the same time. Alternatively, R¹² and R¹³ may be removed after removing R⁹ or CH-N(R¹⁰)(R¹¹). Alternatively, R⁹ or CH-N(R¹⁰)(R¹¹) may be removed after removing R¹² and R¹³.

The condition for removing the protective group, the solvent to be used, and the reaction temperature can be appropriately selected depending on the type of the protective group to be used.

In one embodiment, in formula (X), R¹ and R² each represents methyl, R³ represents tert-butyl, R¹² and R¹³ each represents 2-cyanoethyl, and R⁸ represents N=CH-N(CH₃)₂. When these protective groups are used, a by-product generated after the removal can be easily separated from the compound represented by formula (IV), and thus, the compound represented by formula (IV) can be successfully purified and isolated. When these protective groups are used, the compound or a salt thereof represented by formula (IV) can be produced by simultaneously removing 2-cyanoethyl and CH-N(CH₃)₂ by alkaline treatment (for example, ammonia water treatment, methylamine solution treatment, aqueous solution treatment of a metal hydroxide such as sodium hydroxide, potassium hydroxide, or lithium hydroxide, and aqueous solution treatment of a carbonate such as sodium carbonate or potassium carbonate).

All documents cited herein are incorporated herein by reference in their entirety.

Examples of the present invention described below are intended only to exemplify the present invention rather than to limit the technical scope of the present invention. The technical scope of the present invention is limited only by claims. The present invention can be changed, for example, the constituent components of the present invention can be added, deleted, and replaced without departing from the spirit of the present invention.

### EXAMPLES

### EXAMPLE 1

### Production of tetrakis(2-cyanoethyl)2'-O-(tert-butyldimethylsilyl)-2-N-(dimethylaminomethylene)guanosine3',5'-diphosphate (1):

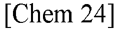

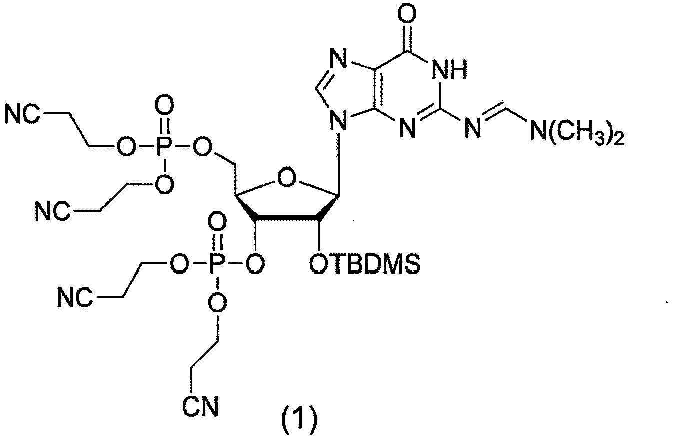

With pyridine four times and toluene four times, 2'-O-(tert-butyldimethylsilyl)-2-N-(dimethylaminomethylene)guanosine (4.0 g, 8.8 mmol) (produced by a method described in Chin, S. A.; Tan, W. J.; Chua K. L.; Lam, Y. Bioorg. Med. Chem. 2010, 18, 6657-6665) was azeotropically dried. The residue was dissolved in acetonitrile (100 mL) and, after 1H-tetrazole (1.4 g, 19.4 mmol) and bis(2-cyanoethyl)N,N-diisopropyl phosphoramidite (5.1 mL, 19.4 mmol) were added thereto, stirred at room temperature for 30 minutes. The solvent was removed under reduced pressure, and the residue was diluted with dichloromethane and washed with saturated sodium bicarbonate water (50 mL) three times and saturated salt water (50 mL) one time. The organic layer was dried with anhydrous sodium sulfate, the solid was filtered, and then, the filtrate was concentrated under reduced pressure. The residue was dissolved in acetonitrile (100 mL) and cooled to 0°C, and then, 5.5 M t-BuOOH (6.4 mL, 35.2 mmol) was added thereto. The reaction system was brought back to room temperature and stirred for 30 minutes, and then, the solvent was removed under reduced pressure. The residue was purified by silica gel column chromatography, and 5.2 g (71%) of the title compound was obtained.
¹H NMR (400 MHz, DMSO-d₆) δ 11.4 (s, 1H), 8.53 (s, 1H), 8.02 (s, 1H), 5.90 (d, J=6.6 Hz, 1H), 5.09 (m, 1H), 5.06 (m, 1H), 4.44 (m, 1H), 4.41-4.33 (m, 2H), 4.26 (m, 4H), 4.22-4.18 (m, 2H), 4.16-4.12 (m, 2H), 3.17 (s, 3H), 3.03 (s, 3H), 2.99-2.96 (m, 4H), 2.92 (t, J=5.8 Hz, 2H), 2.88-2.86 (t, J=5.8 Hz, 2H), 0.72 (s, 9H), -0.03 (s, 3H), -0.20 (s, 3H).
¹³C NMR (101 MHz, DMSO-d₆) δ 158.2, 158.0, 157.8, 150.4, 137.3, 120.5, 119.3, 119.2, 119.1, 86.9, 58.2, 58.1, 58.0, 57.9, 57.7, 57.6, 41.3, 35.2, 25.9, 20.1, 18.1, -4.5, -5.0.
³¹P NMR (162 MHz, DMSO-d₆) δ -1.04, -1.27.
HRMS (ESI-TOF) calcd for C₃₁H₄₆N₁₀O₁₁P₂SiNa [M+Na] 847.2490, found 847.2502.

### EXAMPLE 2

### Production of 2'-O-(tert-butyldimethylsilyl)-guanosine3',5'-diphosphate 2triethylammonium salt (2):

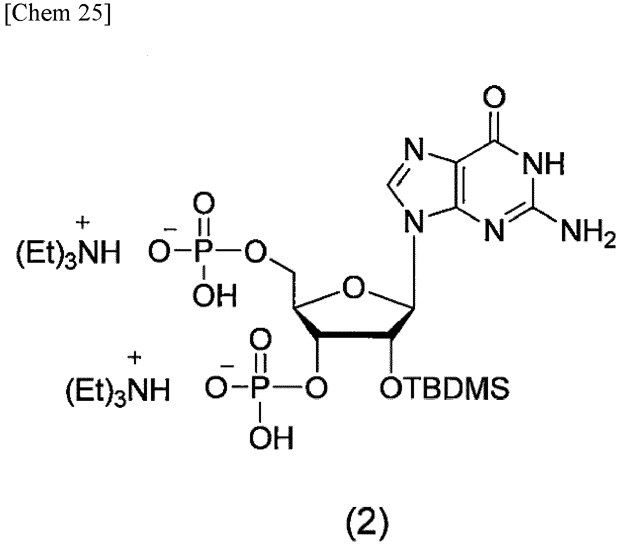

In 28% ammonia water (1 mL), tetrakis(2-cyanoethyl)2'-O-(tert-butyldimethylsilyl)-2-N-(dimethylaminomethylene)guanosine3',5'-diphosphate (20 mg, 20 µmol) produced in Example 1 was dissolved and reacted at 50°C for 13 hours. The solvent was removed under reduced pressure, and the residue was dissolved in a 0.1 M triethylammonium acetate buffer solution and eluted by 2% triethylamine (ethanol: water, 7:3, v/v) filled in a reverse-phase cartridge column to obtain the objective substance at a yield of 62%.
¹H NMR (400 MHz, D₂O) δ 8.05 (s, 1H), 5.77 (d, J=7.6 Hz, 1H), 4.59 (s, 1H), 4.58 (s, 1H), 4.51 (s, 1H), 4.01 (m, 2H), 0.49 (s, 9H), -0.16 (s, 3H), -0.26 (s, 3H).
¹³C NMR (101 MHz, D₂O) δ 158.6, 154.0, 151.8, 137.0, 115.7, 86.2, 83.9, 75.4, 73.8, 64.3, 25.0, 17.4, -5.6, -6.3.
³¹P NMR (162 MHz, D₂O) δ 1.26, -0.06.
HRMS (ESI-TOF) calcd for C₁₆H₂₉N₅O₁₁P₂Si⁻ [M-H] 556.1035, found 556.1022.

### EXAMPLE 3

### Production of 2'-O-(tert-butyldimethylsilyl)-guanosine3',5'-di(dibenzyl pyrophosphate) (3):

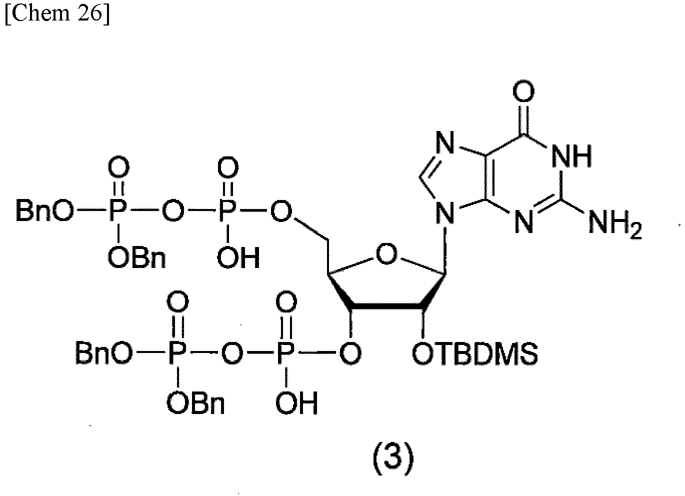

In pyridine (2 mL), 2'-O-(tert-butyldimethylsilyl)-guanosine3',5'-diphosphate 2triethylammonium salt (117 µmol) produced in Example 2 was dissolved, and tri(n-butyl)amine (56 µL, 234 µmol) and DBU (35 µL, 234 µmol) were added thereto. The solvent was removed under reduced pressure, and N,N-dimethylformamide (2 mL) and acetonitrile (1.2 mL) were added to the residue. To this solution, dibenzyl N,N-diisopropyl phosphoramidite (157 µL, 468 µmol) and 5-ethylthiotetrazole (62 mg, 468 µmol) were added, and the solution was stirred at room temperature for one hour. After 5.5 M t-BuOOH (340 µL, 1.87 mmol) was added thereto, the solution was stirred at room temperature for 30 minutes. The reaction solution was concentrated, and the residue was purified by silica gel column chromatography to obtain the objective substance (80%).
¹H NMR (400 MHz, DMSO-d₆-MeOD-d₃) δ 8.11 (s, 1H), 7.30 (m, 20H), 5.89 (d, J=8.0 Hz, 1H), 5.06 (m, 8H), 4.85 (m, 1H), 4.80 (d, J=8.0 Hz, 1H), 4.71 (m, 1H), 4.18 (m, 2H), 0.65 (s, 9H), -0.03 (s, 3H), -0.19 (s, 3H).
³¹P NMR (162 MHz, DMSO-d₆-MeOD-d₃) δ -12.5 (d, J=18.1 Hz, 1P), -12.9 (d, J=18.9 Hz, 1P), -13.1 (d, J=18.9 Hz, 1P), -13.8 (d, J=18.1 Hz, 1P).
HRMS (ESI-TOF) calcd for C₄₄H₅₄N₅O₁₇P₄Si⁻ [M-H] 1076.2240, found 1076.2209.

### EXAMPLE 4

### Production of 2'-O-(tert-butyldimethylsilyl)-guanosine3',5'-dipyrophosphate (4):

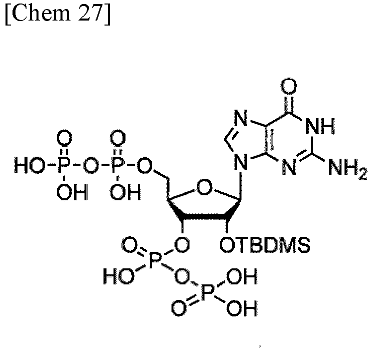

In methanol (1 mL), 2'-O-(tert-butyldimethylsilyl)-guanosine3',5'-di(dibenzyl pyrophosphate) (71 mg, 40 µmol) produced in Example 3 was dissolved, and 10 wt% Pd/C was added thereto. This suspension was stirred at room temperature for four hours under a hydrogen atmosphere. Four hours later, the catalyst was removed by filtration, the solvent was removed under reduced pressure, the residue was dissolved in MeOH (1 mL), and an acetone solution of 0.6 M NaClO₄ (10 mL) was added thereto. This suspension was centrifuged at 4°C for one hour, and then, the solvent was removed. In methanol-water (1:1, v/v, 2 mL), 25 µmol in the precipitate was dissolved, and 10 wt% Pd/C was added thereto. This suspension was stirred at room temperature for one hour under a hydrogen atmosphere. The solvent was removed to obtain the objective substance (51.262 abs in 3mL water, 13.4 µmol, 34%).
¹H NMR (400 MHz, D₂O) δ 8.06 (s, 1H), 5.87 (d, J=5.0 Hz, 1H), 4.24 (m, 1H), 4.16 (m, 2H), 0.56 (s, 9H), -0.05 (s, 3H), -0.10 (s, 3H).
³¹P NMR (162 MHz, D₂O) δ -10.1 (m, 2P), -11.3 (m, 1P), -12.0 (m, 1P).
HRMS (ESI-TOF) calcd for C₁₆H₃₀N₅O₁₇P₄Si⁻ [M-H] 716.0362, found 716.0348.

### EXAMPLE 5

### Production of guanosine3',5'-dipyrophosphate (5)

In an 8% acetic acid aqueous solution, 2'-O-(tert-butyldimethylsilyl)-guanosine3',5'-di(dibenzyl pyrophosphate) produced in Example 4 was dissolved, and the solution was left for 24 hours. The objective substance was confirmed in the reaction solution.

HRMS (ESI-TOF) calcd for C₁₀H₁₆N₅O₁₇P₄⁻ [M-H] 601.9497, found 601.9512.

### EXAMPLE 6

### Production and isolation of guanosine3',5'-dipyrophosphate (5)

In an 8% acetic acid aqueous solution (1 mL), 2'-O-(tert-butyldimethylsilyl)-guanosine3',5'-di(dibenzyl pyrophosphate) (4.76 µmol) produced in Example 4 was dissolved, and the solution was left for 6 hours. The reaction progress was tracked by reverse-phase HPLC (device name: e2695 manufactured by Nihon Waters K.K.; column: XBridge (trademark) C18 manufactured by Nihon Waters K.K. (5 µm, 4.6 × 150 mm); UV detector: Waters 2998; detection at wavelength of 260 nm). After the reaction progress, the reaction was terminated by adding sodium bicarbonate (1.4 mmol, 118.4 mg) to neutralize. A crude product was isolated by reverse-phase HPLC (device name: UV-4070 manufactured by JASCO Corporation; column: XBridge (trademark) BEH C18 OBD Prep (trademark) manufactured by Nihon Waters K.K. 5 µm, 10 × 250 mm) using 100 mM hexafluoro2-propanol and an 8 mM triethylamine buffer solution to obtain the objective substance (1.21 µmol, 25%).
¹H NMR (500 MHz, D₂O) δ 8.14 (s, 1H, H8ₐᵣₒₘ), 6.00 (d, J=6.8 Hz, 1H, H1'), 5.00-4.96 (m, 1H, H3'), 4.92-4.89 (m, 1H, H2'), 4.59-4.58 (m, 1H, H4'), 4.26-4.20 (m, 2H, H5' and H5").
³¹P NMR (202 MHz, D₂O) δ -6.91 (d, J=19.9 Hz, 1P), -8.35 (d, J=20.2 Hz, 1P), -10.37 (t, J=21.3 Hz, 2P).
HRMS (ESI-TOF) calcd for C₁₀H₁₆N₅O₁₇P₄⁻ [M-H] 601.9497, found 601.9512.

### INDUSTRIAL APPLICABILITY

The present invention can be suitably used for efficient production of ppGpp.

## Claims

1. A production method of a compound or a salt thereof represented by formula (I): 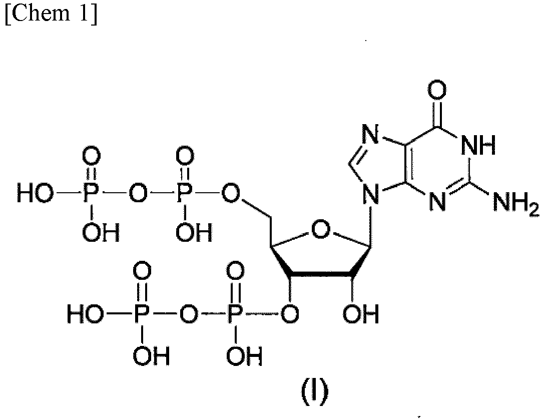 , the production method comprising:
a step of removing Si(R¹)(R²)(R³), and R⁴ and R⁵ from a compound or a salt thereof represented by formula (II): 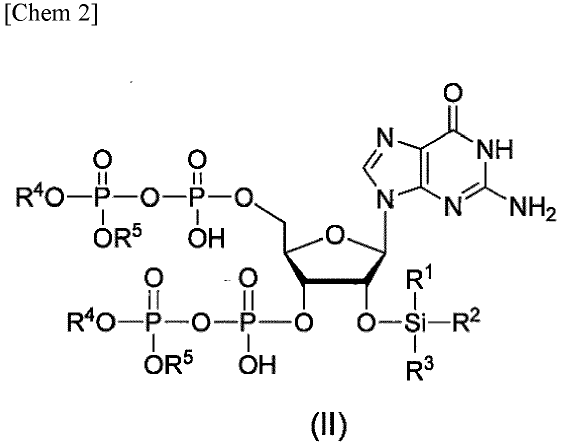 wherein
R¹ to R³ each independently represents C₁₋₁₀ alkyl or C₄₋₁₀ cycloalkyl, and
R⁴ and R⁵ each independently represents a protective group.

2. The production method according to claim 1, wherein the step of removing Si(R¹)(R²)(R³), and R⁴ and R⁵ comprises:
a step of removing R⁴ and R⁵ from the compound or a salt thereof represented by formula (II) to obtain a compound or a salt thereof represented by formula (III): 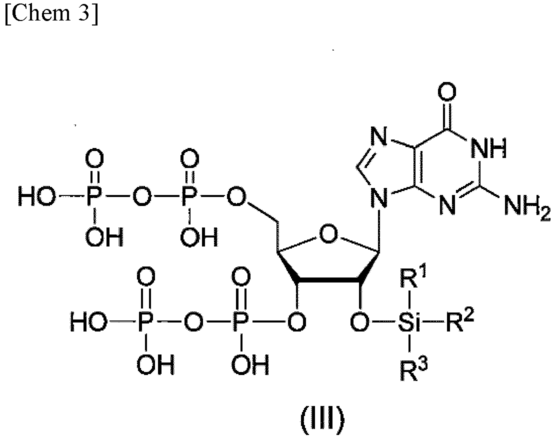 wherein
R¹ to R³ each independently represents C₁₋₁₀ alkyl or C₄₋₁₀ cycloalkyl; and
a step of removing Si(R¹)(R²)(R³) from the compound or a salt thereof represented by formula (III) to obtain the compound or a salt thereof represented by formula (I).

3. The production method according to claim 1 or 2, further comprising:
a step of reacting a compound or a salt thereof represented by formula (IV): 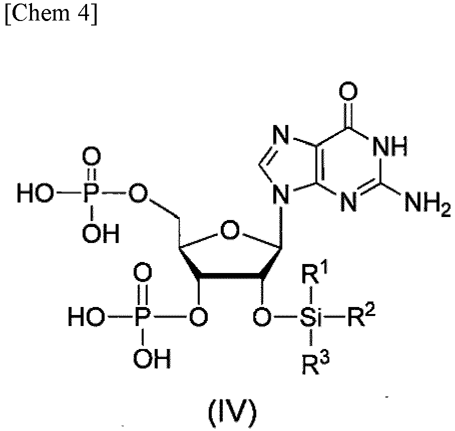 wherein
R¹ to R³ each independently represents C₁₋₁₀ alkyl or C₄₋₁₀ cycloalkyl,
with a phosphoramidite represented by formula (V): 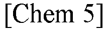 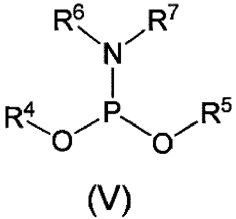 wherein
R⁴ and R⁵ each independently represents a protective group, and
R⁶ and R⁷ each independently represents C₁₋₁₀ alkyl or C₄₋₁₀ cycloalkyl or forms a pyrrolidine ring, a piperidine ring, or a morpholine ring with an adjacent nitrogen atom,
in the presence of an activator to obtain a compound or a salt thereof represented by formula (VI): 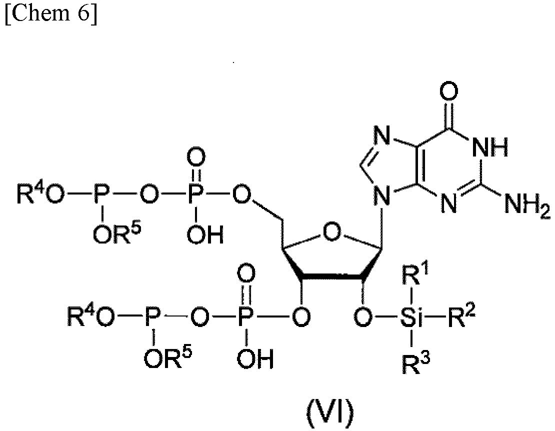 and
a step of oxidizing the compound or a salt thereof represented by formula (VI) to obtain the compound or a salt thereof represented by formula (II).

4. The production method according to any one of claims 1 to 3, wherein R¹ and R² each represents methyl, R³ represents tert-butyl, and R⁴ and R⁵ each represents benzyl.

5. The production method according to any one of claims 1 to 4, further comprising:
a step of reacting a compound or a salt thereof represented by formula (VII): 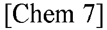 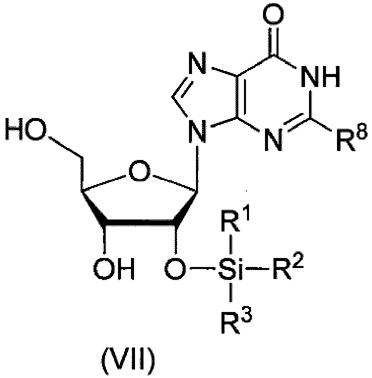 wherein
R¹ to R³ each independently represents C₁₋₁₀ alkyl or C₄₋₁₀ cycloalkyl,
R⁸ represents NHR⁹ or N=CH-N(R¹⁰)(R¹¹),
R⁹ represents a protective group that can be removed under a basic condition, a protective group that can be removed under a reductive condition, or a protective group that can be removed by a light reaction, and
R¹⁰ and R¹¹ each independently represents C₁₋₁₀ alkyl or C₄₋₁₀ cycloalkyl, with a phosphoramidite represented by formula (VIII): 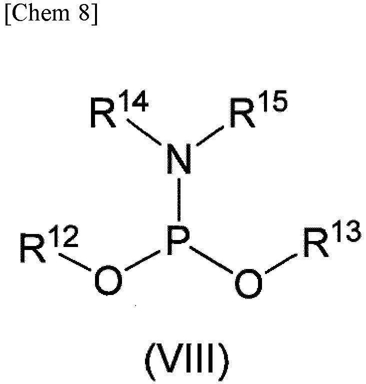 wherein
R¹² and R¹³ each independently represents a protective group that can be removed under a basic condition, a protective group that can be removed under a reductive condition, or a protective group that can be removed by a light reaction, and
R¹⁴ and R¹⁵ each independently represents C₁₋₁₀ alkyl or C₄₋₁₀ cycloalkyl or forms a pyrrolidine ring, a piperidine ring, or a morpholine ring with an adjacent nitrogen atom,
in the presence of an activator to obtain a compound or a salt thereof represented by formula (IX): 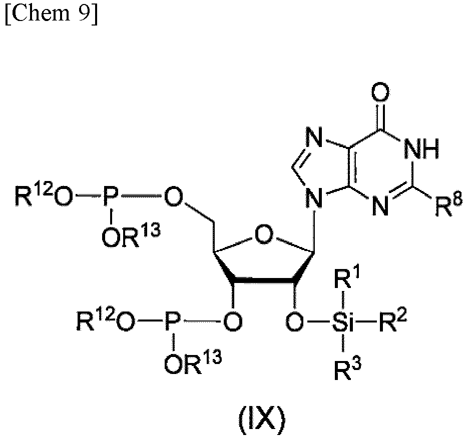
a step of oxidizing the compound represented by formula (IX) to obtain a compound or a salt thereof represented by formula (X): 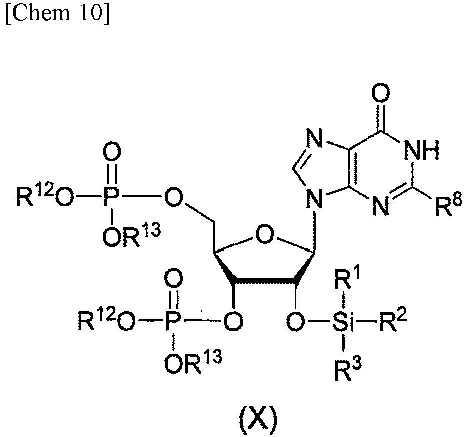 and
a step of removing R⁹ or CH-N(R¹⁰)(R¹¹), and R¹² and R¹³ from the compound represented by formula (X) to obtain the compound or a salt thereof represented by formula (IV).

6. The production method according to claim 5, wherein R¹² and R¹³ each represents 2-cyanoethyl, and R⁸ represents N=CH-N(CH₃)₂.

7. A compound or a salt thereof represented by formula (II): 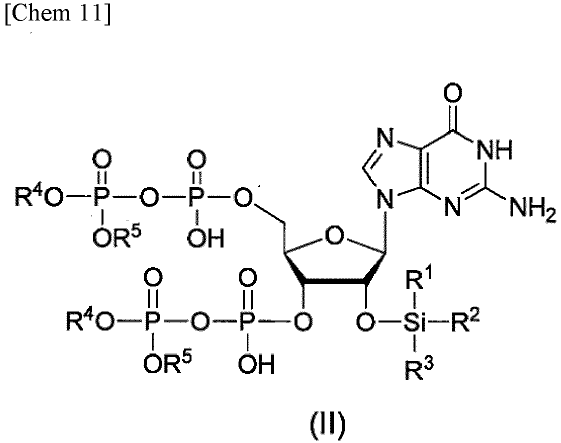 wherein
R¹ to R³ each independently represents C₁₋₁₀ alkyl or C₄₋₁₀ cycloalkyl, and
R⁴ and R⁵ each independently represents a protective group.

8. A compound or a salt thereof represented by formula (III): 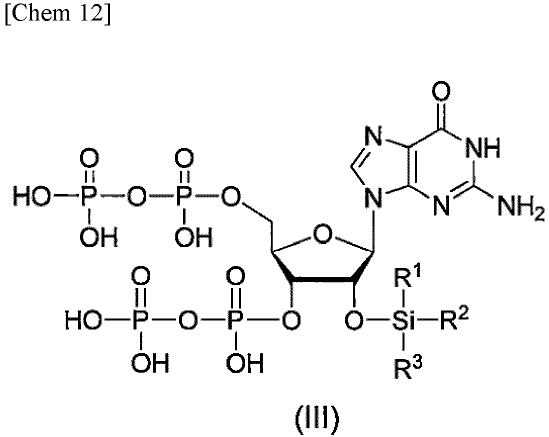 wherein
R¹ to R³ each independently represents C₁₋₁₀ alkyl or C₄₋₁₀ cycloalkyl.

9. A compound or a salt thereof represented by formula (IV): 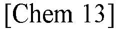 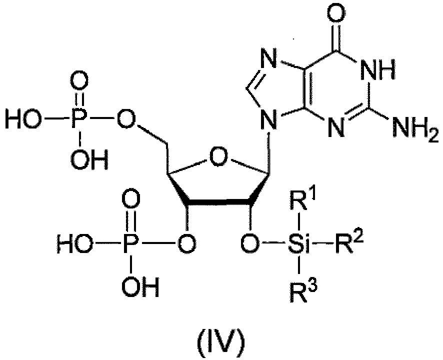 wherein
R¹ to R³ each independently represents C₁₋₁₀ alkyl or C₄₋₁₀ cycloalkyl.
